# EUROPEAN PATENT SPECIFICATION

(11) **EP 0 906 465 B1**
(45) Date of publication and mention of the grant of the patent: **18.10.2000**
(21) Application number: 97931197.4
(22) Date of filing: 17.06.1997
(51) Int. Cl.: D21C 5/02, C07C 51/41

(54) **DEINKING PROCESS**
VERFAHREN ZUM DEINKEN VON ALTPAPIER
PROCEDE DE DESENCRAGE

(30) Priority: 20.06.1996 US 666901
(43) Date of publication of application: 07.04.1999
(73) Proprietor: High Point Chemical Corporation, High Point, NC 27260 (US)
(72) Inventor: ROBINSON, Peter, M., High Point, NC 27262 (US); BROOKS, Robert, E., High Point, NC 27265 (US); SINGLETON, Terry, E., Jamestown, NC 27282 (US)
(74) Representative: Des Termes, Monique
(86) International application number: US9710396
(87) International publication number: WO9748850

(56) References cited:
- EP-A- 0 335 260
- WO-A-94/28238
- DD-A- 106 629
- DATABASE WPI Section Ch, Week 8225 Derwent Publications Ltd., London, GB; Class E12, AN 82-51289E XP002042432 & JP 57 077 642 A (KINDAI KAGAKU KK) , 15 May 1982

## Description

### FIELD OF THE INVENTION

The present invention is directed to an improved deinking process, preferably flotation deinking, which entails the use of an alkaline earth metal tallate, more specifically a calcium tallate, which has been prepared by a double displacement method. The process exhibits improved deinking performance, as compared to an equivalent amount of either (i) a tallate prepared in situ by the addition of tall oil fatty acids to hard water containing large amounts of calcium, or (ii) a conventionally prepared calcium salt of a fatty acid, e.g. calcium stearate.

### BACKGROUND OF THE INVENTION

Deinking is the method of removing ink and other contaminants from waste paper. There are two main techniques in current use: "flotation" and "wash" deinking. In "flotation deinking," an aqueous suspension of waste paper pulp fibers, ink, and other non-cellulosic contaminants is formed and then air is mixed into the suspension to cause the ink to "float." In the presence of certain additives, air bubbles selectively attach to ink particles and carry those particles to the surface of the aqueous suspension thereby forming an ink rich froth. After the froth is removed, a relatively ink-free fiber slurry remains.

The conventional industry chemical formulations for flotation deinking for many years have entailed using a fatty acid or fatty acid soap. See, for example, U.S. Patent Nos. 4,964,949 and 4,483,741. Fatty acids and fatty acid soaps have a number of problems associated with their use. They require high dosage rates, typically at least about 8 kg/1,000 kg (16 pounds/ton) of waste paper (0.8 wt %) but often as high as 15 kg/1000 kg ((30 pounds/ton) (1.5 wt %). Also, despite their use, fatty acids and fatty acid soaps are relatively weak deinking agents especially in the presence of hydrophilic flexographic inks.

Conventional methods for preparing alkaline earth metal tallates have entailed a single reaction between an alkaline earth metal hydroxide, e.g. calcium hydroxide, and tall oil fatty acids (TOFA). Oftentimes, this has been done in situ during a deinking process by the addition of unneutralized tall oil fatty acids to water having high hardness, c.f. U.S. Pat. No. 5,417,-807. When the water hardness is too low, sufficient calcium ions can be provided by the addition of a salt such as calcium chloride to the water along with the tall oil fatty acids.

Calcium stearate salts are known deinking agents which are conventionally prepared by combining calcium hydroxide and stearic acid under controlled conditions of temperature and mixing.

It has now been unexpectedly discovered that the use of a alkaline earth metal tallate dispersion which has been prepared by a double displacement method prior to its addition to the printed media being deinked produces improved deinking performance, particularly in flotation deinking. The deinking dispersions of this invention are disclosed in co-pending application, WO 9 748 849, (Attorney Docket No. HPCC 104), filed on the same date herewith.

Accordingly, it is an object of the present invention to develop an improved process for removing inks from printed media, preferably recycled printed media.

### SUMMARY OF THE INVENTION

The present invention is directed to a process for removing ink from printed media, preferably recycled printed media, by (i) forming a pulp slurry comprising printed media and water, (ii) adding an alkaline earth metal tallate dispersion to the slurry; and (iii) subjecting the slurry to flotation deinking in a flotation cell, wherein the alkaline earth metal tallate dispersion has been prepared by a double displacement method prior to being added to the slurry. Preferably the tallate is calcium tallate. Also preferably, the tallate is added in an amount sufficient to increase the brightness of a filter pad prepared from the printed media by least about 1 TAPPI brightness unit as compared to an equivalent amount of either (i) a tallate prepared in situ by the addition of tall oil fatty acids to water containing calcium ions, or (ii) a conventionally prepared calcium salt of a fatty acid, e.g. calcium stearate.

### DETAILED DESCRIPTION OF THE INVENTION

In the deinking process of the present invention, a flotation stage is used to remove inks from printed media. Generally, the process comprises a pulping step, wherein printed waste paper, often in combination with virgin wood pulp, is treated in an alkaline medium with water in a reactor/pulper having an agitation system. The aqueous suspension so formed contains pulp fiber, inks, coatings, inorganic fillers, and the like, generally in an amount of from about 3 to 18 wt % and is maintained at an alkaline pH, generally at about 7.5 to 11. The pulp slurry may contain any type of printed media, such as newspapers, magazines, printed cardboard, colored printed media, and the like. Typical chemicals used in the pulping stage include such as NaOH and hydrogen peroxide. Sodium silicate, a metal chelating agent such as diethylenetriaminepentaacetic acid (DTPA), and calcium chloride may also be added to the pulper. A non-ionic surfactant is commonly added directly to the pulper during the pulping step. Further, surfactant may also be added to the flotation cells (a split addition technique) to enhance performance.

After the slurry exits the pulper, it is diluted to about 1 wt % solids and an alkaline earth metal tallate which has been previously prepared by the double displacement method (described below) is added thereto and mixed therein. Generally, the addition and mixing will be performed within the pipeline feeding the diluted pulp slurry into the flotation cells or immediately upon entering said equipment. Alternatively, the tallate may be added partially during the pulping operation and partially upon entry to flotation. Thereafter, air is introduced into the flotation equipment to cause vigorous mixing of the diluted pulp slurry. The water in the flotation slurry may be of any hardness, including below 50 ppm Ca.

Filter pads prepared from pulps deinked with a tallate in accordance with the present invention are significantly brighter than equivalent pads prepared with either (i) a tallate prepared in situ by the addition of tall oil fatty acids to water containing calcium, or (ii) a conventionally prepared calcium salt of a fatty acid. The process also increases the ink removal rate over conventional processes which utilize calcium stearate and/or calcium stearate dispersions, thereby allowing increased production rates without sacrificing product quality.

The double displacement method used to prepare the alkaline earth metal tallate dispersion of the present invention is performed by (i) forming a pre-emulsion of a tall oil fatty acid, water, and a surfactant; (ii) forming separately an alkaline earth metal salt dispersion from an alkaline earth metal oxide or hydroxide, an organic acid having a pKa ranging from about 2 to about 6, and water; and (iii) incorporating the pre-emulsion into the alkaline earth metal salt dispersion under conditions of high shear and low pressure. More particularly, the pre-emulsion is formed from those tall oil fatty acids present in partially de-rosinated tall oil, water, and a surfactant. Also more particularly, the organic acid is a lower carboxylic acid.

The term "tall oil fatty acids" in this application refers to the mixture of fatty acids which are naturally occurring in tall oil. More specifically, the acids are generally of the formula R⁵COO-M, wherein R⁵ is a linear, branched, or cyclic alkyl or alkenyl group having about 14 to about 20 carbon atoms and wherein M is hydrogen or a counter-ion such as K, Na or Ca. Tall oil fatty acids are generally present in the pre-emulsion in an amount from about 15 to 45 wt %, preferably from about 20 to 40 wt %, and more preferably from about 25 to 30 wt %, based upon the total weight of the desired final product. The primary acids in the tall oil fatty acids have been reported to be about 45 wt % oleic acid, about 40 wt % linoleic acid, and up to 15 wt % rosin acid. Preferably, the tall oil fatty acids are obtained by partially derosinating tall oil in a conventional manner.

The surfactant used to form the pre-emulsion is any surfactant which is capable of decreasing the interfacial tension between the oil (fatty acids) and water sufficiently that a stable pre-emulsion can form. While any conventional surfactant may be used, it is preferable that the surfactant be a non-ionic alkoxylated compound represented by the formula:

RO[(AO)ₙ-R^{o}]ₘ

wherein R is selected from the group consisting of (a) linear and branched alkyl and alkenyl groups having about 8 to about 22 carbon atoms; (b) R¹CO- wherein R¹ is a linear or branched alkyl or alkenyl group having about 7 to about 24 carbon atoms; (c) (R²)ₐC₆H₍₅₋ₐ₎- wherein R² is a linear or branched alkyl and alkenyl group having about 7 to 24 carbon atoms and "a" is 0 or 1; (d) a group derived from a linear, branched, and cyclic aliphatic polyol having from about 2 to 14 carbon atoms; and (e) a group derived from a linear, branched, and cyclic aliphatic diacid having about 8 to 20 carbon atoms; AO is an oxyalkylene group having 2 to about 4 carbon atoms or a mixture of such groups in random or block configuration; R^{O} is selected from the group consisting of H, R¹ and R¹CO; n is a number from about 5 to about 150; and m is an integer from about 1 to 6.

Suitable polyols useful for preparing the preferred surfactant include, for example, ethylene glycol, propylene glycol, trimethylene glycol, butylene glycol, glycerol, trimethylol propane, trimethylol ethene, 1,2,4-butanetriol, 1,2,6-hexanetriol, 1,1,1-trimethylol hexane, pentaerythritol, diglycerol, and sorbitol. Suitable diacids include, for example, octanedioic (suberic), nonanedioic (azelaic), hexadecanedioic (thapsic), octadecanedioic, and heneicosanedioic (japanic) acids.

Such surfactants are commercially available and may be made by techniques well known in the art. Specific examples of such surfactants include (i) the Neodol® surfactants, e.g. Neodol® 1-3, Neodol® 1-5, Neodol® 1-7 and Neodol® 1-9 from Shell Chemical, which are C₁₁ alcohols which have been ethoxylated with 3, 5, 7, and 9 moles of ethylene oxide, respectively; (ii) Kao Emulgen® 150 surfactant with C₁₂ alcohols with 50 moles of ethylene oxide, available from Kao Corporation, Japan; (iii) Hipochem® CO-130, a surfactant containing castor oil with about 130 moles of ethylene oxide, available from High Point Chemical Company, High Point, NC.

More preferably, a binary surfactant system is used. Most preferably, a combination of Neodol® 1-3 and Neodol® 1-7 is used.

The surfactant component is generally used in the present invention in an amount of from about 1 to 10 wt % based on the weight of the pre-emulsion, preferably from about 1.5 to 6 wt %, and more preferably from about 5 to 6 wt %.

In addition to the tall oil fatty acids, surfactant, and water, the pre-emulsion may contain additional materials. For example, the pre-emulsion may contain rosin acids, i.e. alkylated, tricyclic, unsaturated organic acids, which are naturally present in tall oil. The two main rosin acids in tall oil are abietic acid and pimaric acid. Generally, the pre-emulsion will contain rosin acids in an amount ranging from less than about 1 wt % to about 15 wt %, more preferably about 5 to about 10 wt %, and even more preferably about 7.5 wt % to about 10 wt %.

Furthermore, the pre-emulsion may also contain an anionic surfactant such as dodecylbenzene sulfonate, fatty acid products such as Diacid 1575 or Diacid 1550 (combinations of C₁₈-monomer fatty acid, C-₂₁ fatty acid lactone, and C₃₆ dimer fatty acid) available from Westvaco Chemicals and stearic acid, where amounts of each range from about 1 to 10 wt %, preferably 1 to 5 wt % based on the total weight of the pre-emulsion. Other fatty acids having about 8 to about 22 carbon atoms may be added to optimize the deinking performance of the resulting composition for specific paper mills. Also, monomer acids and dimer acids may be included in the pre-emulsion in amounts ranging up to about 50 wt % of the tall oil fatty acids in the pre-emulsion. Organic acids having between about 16 and about 22 carbon atoms, may also be present.

The pre-emulsion is formed by mixing tall oil fatty acids, the surfactant (or surfactants), water, and any other suitable materials until a substantially homogenous pre-emulsion is formed. Any suitable mixing device may be used provided that it can form a pre-emulsion. Preferably, a high shear, low pressure Silverson in-line mixer (homogenizer), available from Silverson Machines Inc., East Longmeadow, Massachusetts, is used. When the pre-emulsion ingredients are mixed in such a mixer, they rotate intensely so that a suction results and liquid and solid materials are drawn upwards from the bottom of the mixing vessel. A rotor, typically consisting of three or four sets of finely machined teeth that run concentrically against three or four sets of stators, subjects the ingredients to millions of individual shearing actions per second, providing intense shear. The Silverson mixer appears to generate less heat and a smaller heat rise than do many other mixers. It is believed that the low generation of heat during the mixing may be beneficial to preparing the stable alkaline earth metal tallate dispersions of this invention.

The pre-emulsion so formed is inherently stable, i.e. it is sufficiently stable that it can be prepared and stored for up to about 10 days before being used to form the alkaline earth metal tallate dispersion. When stored prior to use, the pre-emulsion is preferably maintained at a temperature of about 15 to about 30°C.

The salt dispersion is a highly alkaline aqueous dispersion prepared from an acid and a base which, when combined with the pre-emulsion according to the specific manner of this invention, forms the improved alkaline earth metal tallate dispersions of this invention. The acids useful for preparing the salt dispersion are weak acids having a pKa value of about 2 to less than 6. Suitable such acids include the lower carboxylic acids, e.g. formic acid, hydroxy acetic acid, peracetic acid, propionic acid, butyric acid, and the like. Acetic acid, is the currently preferred lower carboxylic acid. Suitable acids also include chloro acids such as chloroacetic acid, 2-chloropropionic acid, 3-chloropropionic acid, and 4-chlorobutyric acid. For example, the following acids have the indicated corresponding pKa values: formic acid (3.77), acetic acid (4.76), propionic (4.88), butanoic acid (4.82), nonanoic acid (4.95), chloroacetic acid (2.86), 2-chlorobutyric acid (2.84), 3-chlorobutyric acid (4.06), 4-chlorobutyric acid (4.52), hydroxy acetic acid (3.83), malonic acid (2.83), phenyl acetic acid (4.31), vinyl acetic acid, or 3-butanoic acid (4.35). Preferably, the acids contain about 12 carbon atoms or less. More preferably, the acids contain less than 10 carbon atoms. Generally, the amount of acid added to form the salt dispersion is from about 0.5 to about 1.0 wt %, based on the weight of the salt dispersion.

An alkaline earth metal oxide or hydroxide is used as the base. The preferred bases are hydrated lime and calcium oxide. The base is present in excess of the stoichiometric amount required to neutralize the acid, preferably in at least 25 % excess, more preferably at least 100% excess, and most preferably at least about 1000 % excess.

The resulting salt dispersion thus contains salts containing an alkaline earth metal. Examples of such salts include calcium acetate, calcium propionate, calcium butyrate, magnesium acetate, barium propionate, and the like. The salt dispersion generally has a pH of about 12 or higher.

The salt dispersion is formed by mixing the acid and alkaline earth metal oxide or hydroxide, and water by any method which produces a dispersion, i.e. conventional stirring techniques. While the mixing may be performed at any temperature, a temperature that is less than about 55°C, less than about 50°C, or less than about 45°C may be more suitable. Even more preferably, a temperature of less than 40°C, preferably less than about 35°C, and even more preferably less than about 30°C, is preferred since these temperatures permit the salt dispersion to be used immediately upon formation and without cooling.

It is believed that maintaining the temperature below about 40°C during neutralization, high shear mixing (i) minimizes the exposure of the tall oil fatty acid particles to conditions likely to form sticky agglomerates, and (ii) also enhances product fluidity by rapidly cooling the salt dispersion as quickly as it is formed.

To complete the manufacture of the alkaline earth metal tallate dispersion, a pre-emulsion as described above is incorporated into a salt dispersion with mixing (homogenization) being performed under conditions of high shear and low pressure. The shear must be sufficiently high and the pressure sufficiently low to generate sufficient turbulence to produce a stable alkaline earth metal tallate dispersion. A suitable mixing device is a high shear low pressure mixer such as the Silverson in-line mixer (homogenizer) described previously. The high shear, low pressure mixing continues until a uniform alkaline earth metal tallate dispersion forms. The maximum temperature to which the system is exposed during the mixing should be less than about 55°C, preferably less than about 50°C, and even more preferably less than about 45°C. The mixing is performed at a sufficiently low rate of addition and pressure that the temperature does not exceed the temperature maximum. Generally, this can be accomplished by starting with a pre-emulsion at room temperature and then using a mixing pressure of below about 137.9 kPa (20 psig), more preferably below about 103.4 kPa (15 psig). The mixing continues for a limited time to minimize the formation of sticky agglomerates, contaminant gels, and the like, which cause product degradation and reduce product stability. The high shear causes the sizes of the pre-emulsion particles to be reduced sufficiently to form the stabilized alkaline earth metal tallate dispersions. Preferably, the high shear results in the formation of a bimodal particle size distribution, i.e. a particle size distribution containing two clear maxima, one in the range of about 10 to about 30 microns and a second from about 100 to about 150 microns. Preferably, the particle size maxima are in the ranges of (i) from about 15 to about 25 microns and (ii) from about 100 to about 125 microns.

The double displacement method used to prepare the calcium tallate yields a product having good long term stability, i.e. 4 to 6 months at a maximum temperature up to 30°C, without post-formation stabilizer addition.

When it is expected that the alkaline earth metal tallate dispersions may be exposed to elevated temperatures during storage prior to use, e.g. during summer, post-formation stabilization is recommended. Suitable stabilizers are anionic, cationic, or non-ionic surfactants. Examples of specific such post-formation stabilizers include dicarboxylic acids, sulfonates, ether carboxylates, quaternary ammonium compounds, and EO:PO block and random polymeric surfactants. Specific currently preferred post-formation stabilizers include dodecylbenzene sulfonate, the dicarboxylic acid Diacid 1575 and a sulfonated naphthalene-formaldehyde condensate, Daxad 16 (Hampshire Chemical, Lexington, MA as well as ether carboxylates NEODOX® 91-5, NEODOX® 91-7, NEODOX® 1-4, NEODOX® 23-4, NEODOX® 23-6, NEODOX® 25-6, and NEODOX® 25-11, NEODOX® 45-7 from Shell Chemical, and benzyl trimethyl ammonium chloride. The post-formation stabilizers are generally used in an amount of about 0.5 to 2.5 wt % based on the weight of the alkaline earth metal tallate dispersion, preferably in an amount of about 0.5 to about 1 wt % thereof. The stabilizers may be incorporated into a previously prepared dispersions by any conventional mixing equipment. The stabilizers have been found to increase the stability of the dispersions at maximum temperatures of about 45°C to about 50°C from less than about 10 days to more than 90 days.

Although not necessary, still further deinking has been found to occur when an additional non-ionic or anionic surfactant is added to the pulp slurry. Preferably, the surfactant is a non-ionic surfactant. More preferably, the non-ionic surfactant is a non-ionic alkoxylated surfactant, which may be represented by the general formula:

R-O-[(AO)ₙ-R^{o}]ₘ

wherein AO is an oxyalkylene group having 2 to about 4 carbon atoms or a mixture of such groups in random or block configuration; R is selected from the group consisting of (a) linear and branched alkyl and alkenyl groups having about 7 to about 24 carbon atoms; (b) R¹CO- wherein R¹ is a linear or branched alkyl or alkenyl group having about 7 to about 24 carbon atoms; (c) (R²)ₐC₆H₅₋ₐ- wherein R² is linear or branched C₇-C₁₈ alkyl and alkenyl and "a" is an integer from 1 to 3; (d) a group derived from a linear, branched, and cyclic aliphatic polyol having about 2 to 6 carbon atoms; and (e) a group derived from a linear, branched, and cyclic aliphatic diacid having about 8 to 42 carbon atoms; R^{o} is selected from the group consisting of H, R³ and R³CO; n is a number from about 4 to about 250; and m is an integer from about 1 to 6.

Suitable polyols include, for example, ethylene glycol, propylene glycol, trimethylene glycol, butylene glycol, glycerol, trimethylol propane, trimethylol ethene, 1,2,4-butanetriol, 1,2,6-hexanetriol, 1,1,1-trimethylol hexane, pentaerythritol, diglycerol, and sorbitol. Suitable diacids include, for example, octanedioic (suberic), nonanedioic (azelaic), hexadecanedioic (thapsic), octadecanedioic, and heneicosanedioic (japanic) acids.

The non-ionic surfactant may comprise mixtures of two or more ethoxylated and/or propoxylated materials. Presently preferred non-ionic surfactants include surfactants based on mono- and diglycerides or a naturally occurring fatty acid which have been ethoxylated and/or propoxylated. A particularly preferred non-ionic surfactant is the mixture derived from the reaction of a fat, such as tallow, with potassium hydroxide, glycerol, ethylene oxide and propylene oxide, such as is described in U.S. Pat. Nos. 4,964,949 and 5,100,574 which includes as major components each of the following structures:

C-H₂O-C(O)-R³ C-H₂O-C(O)-R³

C-HO-(AO)ₙ-R⁴ and C-HO-C(O)-R³

C-H₂O-(AO)ₙ-R⁴ C-H₂O-(AO)ₙ-R⁴

wherein R³ is an alkyl group derived from the fat, e.g. tallow, and R⁴ is either H or R¹CO, where R¹ is a linear or branched alkyl or alkenyl group having about 7 to about 24 carbon atoms. The non-ionic surfactant generally functions as a penetrant of the paper fiber in the slurry and stabilizes the ink particles so that they do not redeposit on the pulp fibers. Generally, the non-ionic surfactant is either commercially available or may be made by techniques well known in the art.

The alkaline earth metal tallate dispersion may be added to the pulp slurry continuously or sporadically, depending on the application, until the tallate has been substantially distributed throughout the pulp slurry. The dispersion is added in an amount sufficient to increase the brightness of a filter pad prepared from printed media by least about 1 TAPPI brightness unit. Generally, the dispersion is added in an amount ranging from at least about 0.25 to about 2.5 wt %, more preferably from about .5 to about 2.0 wt %, and even more preferably from about .75 to about 1.0 wt %, based on the weight of the pulp slurry. The actual amounts of alkaline earth metal tallate used in a specific application may be affected by factors such as (i) the specific composition of the pulp slurry, (ii) the type of ink in the printed media, and (iii) the amount of ink in the printed media.

The advantages of the deinking method of the present invention as compared with conventional methods are illustrated in the following examples in which all parts and percents are by weight unless otherwise specified. All filter pads tested for brightness were made using alum as specified in TAPPI Test Method T 218 om-83. Where necessary the pulp samples were first diluted to 1 wt % solids with tap water, 2 ml of 10% alum solution added to each sample, and the slurry then thickened on a Buchner funnel using Ahlstrom grade 631-25 filter paper. The filter pads were air dried before taking brightness measurements. All brightness data was obtained using an ACS Spectrosensor II spectrophotometer and reported as TAPPI 452 brightness. The brightness data represents the percent of light at wavelength 452 nanometers which is reflected off the filter pad and recorded by the spectrophotometer.

### PREPARATION OF A CALCIUM TALLATE DISPERSION

A calcium tallate dispersion was prepared by a double displacement method. First, a pre-emulsion was prepared from 963.9 parts tall oil fatty acid mixture, 837.5 parts water, and a binary surfactant system of 36.1 parts Neodol® 1-3 and 71.8 parts Neodol® 1-7. The ingredients were mixed in a Silverson mixer (Model No 450LS) for about 30 minutes at room temperature.

A calcium acetate salt dispersion was prepared by mixing 11.9 parts of acetic acid, 1316.3 parts of water, and 263.2 parts hydrated lime at 20°C. The resulting dispersion had a beige color and separated upon standing, i.e. without agitation.

The pre-emulsion, which had been prepared the day previously and stored at room temperature, was incorporated into the salt dispersion by continuously adding the pre-emulsion to the stirred and cooled calcium dispersion for about 60 to 75 minutes and subsequently homogenizing by means of a Silverson in-line high shear low pressure homogenizer. The homogenization lasted for a total of about 30 minutes with 5 passes through the homogenizer.

The resulting alkaline earth metal tallate dispersion demonstrated excellent stability at room temperature, desirable particle distributions, and good fluidity. The dispersion was free-flowing, had a bi-modal particle size distribution with a first maxima at about 10 to 30 microns and a second at about 90 to 110 microns, and was stable for up to 180 days, at a temperature of 25-30°C during which time the bi-modal particle size distribution was retained. The initial viscosity was 50 to 100 cps, and the viscosity remained in the range of about 150 to about 300 cps, and did not increase to more than 500 cps.

### EXAMPLE 1

To evaluate the deinking effectiveness of the calcium tallate dispersion prepared above, a pulp slurry was prepared by shredding dry a mixture of 30% old magazines (OMG), 70% old newspapers (ONP) (25% flexo printed) and combining the mixture to form a master batch. The mixture was added to a laboratory pulper along with water (48.9°C) (120°F) to form a pulp slurry having a final consistency of 4.5%. The water contained 1.3 % sodium hydroxide, 1.1% hydrogen peroxide, and 1.8 % sodium silicate, wherein the percentages were calculated on a 100% active basis based on the dry weight of the waste paper added to the pulper. The water had a hardness of 100 ppm. The combination of chemicals, water and waste paper was pulped for 8 minutes. The contents were diluted to 1%, a filter pad (identified as "Fb" for "before flotation") was made from the pulp and the brightness of the pad was measured at this point. The brightness was 44.1 TAPPI units.

The calcium tallate dispersion prepared by the double displacement method described above was added to the slurry. The slurry was placed in a laboratory flotation cell and run for 6 minutes with an air flow of 3 liters per minute. Froth collected on the surface and was removed periodically. The foam height was measured to be 1.5 cm. After the 6 minutes, the air flow to the flotation cell was stopped and the operation ended. A filter pad (identified as "Fa" for "after flotation") was made and the brightness of the pulp was measured. The filter pad (Fa), after flotation, exhibited a brightness of 59.6 TAPPI units.

### EXAMPLE 2

The procedure of Example 1 was repeated except that the water hardness was reduced to 40 ppm Ca. A filter pad, Fb, of the pulp before undergoing flotation exhibited a brightness of 44.5 TAPPI units. The filter pad (Fa) after flotation exhibited a brightness of 54.5 TAPPI units.

### COMPARATIVE EXAMPLE A

To compare the deinking effectiveness of the calcium tallate dispersion used in Examples 1 and 2, a 1.0% calcium stearate salt composition was prepared in a conventional single step reaction by mixing calcium hydroxide and stearic acid in a high speed mixer. The deinking performance of the conventionally-prepared calcium stearate composition was determined as in Examples 1 and 2. The results, along with those of Examples 1 and 2 are shown in Table 1.

**TABLE 1**

| BRIGHTNESS (in TAPPI units) | | | |
|---|---|---|---|
| PROCESS USED | WATER HARDNESS (ppm Ca) | Fb (before flotation) | Fa (after flotation) |
| Invention 1% tallate | 40 | 44.5 | 54.5 |
| Prior art 1% Ca Stearate | 40 | 44.5 | 53.8 |
| Invention 1% tallate | 100 | 44.1 | 59.6 |
| Prior art 1% Ca Stearate | 100 | 44.2 | 58.2 |

The data in Table 1 shows that, at a water hardness of 40 ppm Ca, the present invention produced a pulp that was 0.7 brightness points greater than the pulp produced by the use of the conventionally-prepared calcium stearate, and that at a water hardness of 100 ppm Ca, the present invention produced a pulp that was 1.4 brightness points greater than the pulp produced with the conventionally-prepared calcium stearate.

### EXAMPLE 3

The deinking effectiveness of calcium tallate dispersions prepared by the double displacement method described above and then post-stabilized was determined on a fresh batch of pulp containing 30% OWG and 70% ONP. 1 wt % of each of sodium napthalenesulfonate, dodecylbenzene, sulfonic acid, and dicarboxylic acid (Diacid 1575, available from Westvaco, Inc.) was added to a dispersion. Each of the compositions was stored for 90 days and exhibited a viscosity of less than 500 cps after the 90 days. The compositions were evaluated for deinking in accordance with Example 1 and the results are shown in Table 2.

**TABLE 2**

| Stabilizer | Fb (Brightness Before) | Fa (Brightness After) |
|---|---|---|
| Sodium Napthalene Sulfonate. | 44.1 | 52.6 |
| Dodecylbenzene Sulfonic acid | 44.2 | 52.9 |
| Dicarboxylic acid (Diacid 1575) | 44.0 | 52.5 |

### COMPARATIVE EXAMPLE B

The procedure of Comparative Example A was repeated to compare the deinking effectiveness of (i) calcium tallate of this invention prepared by the double displacement method; (ii) calcium tallate prepared in situ in accordance with U.S. Pat. No. 5,417,807 by adding tall oil fatty acids and calcium (sufficient to produce a water hardness of 300 ppm Ca); (iii) calcium stearate prepared by mixing calcium hydroxide and stearic acid in a high speed mixer. The brightness (in TAPPI units) of filter pads prepared before and after flotation deinking were:

| SAMPLE | Fb | Fa |
|---|---|---|
| | (Brightness Before) | (Brightness After) |
| (i) | 43.8 | 52.0 |
| (ii) | 44.0 | 50.7 |
| (iii) | 43.7 | 48.9 |

The results show that all of the methods utilizing the post-stabilized dispersions of Example 3 performed better than the methods using samples 1, 2 and 3.

## Claims

1. A process for removing inks from printed media which includes steps of: (i) forming a pulp slurry comprising printed media and water, (ii) adding an alkaline earth metal tallate to the pulp slurry in an amount sufficient to remove ink from the slurry and increase the pulp brightness, and (iii) subjecting the pulp slurry to flotation deinking in a flotation cell, characterized in that the alkaline earth metal tallate is a dispersion prepared by a double displacement method prior to its being added to the pulp slurry, wherein the double displacement method entails (a) forming a pre-emulsion of tall oil fatty acids, water, and a surfactant; (b) forming separately an alkaline earth metal salt dispersion from an alkaline earth metal oxide or hydroxide, an organic acid having a pKa ranging from 2 to 6, and water and (c) incorporating the pre-emulsion into the alkaline earth metal salt dispersion under conditions of high shear and low pressure.

2. The process of Claim 1, characterized in that the alkaline earth metal tallate comprises calcium tallate.

3. The process of claim 1, characterized in that the alkaline earth metal tallate is added in an amount of 0.25 to 2.5 wt %.

4. The process of Claim 1, characterized in that the alkaline earth metal tallate is added to the slurry prior to the flotation deinking, or during the flotation deinking, or both prior to and during the flotation deinking.

5. The process of Claim 1 characterized in that the pre-emulsion contains tall oil fatty acids present in partially de-rosinated tall oil.

6. The process of Claim 1, characterized in that the organic acid is a lower carboxylic acid containing 1 to 12 carbon atoms.

7. The process of Claim 1, characterized in that the pre-emulsion contains tall oil rosin acids in an amount ranging up to 10 wt %.

8. The process of Claim 1, characterized in that the pulp slurry comprises an aqueous suspension containing pulp fiber, inks, coatings, and inorganic fillers, in an amount of from 3 to 18 wt % and the pulp slurry is maintained at a pH of 7.5 to 11 during the flotation deinking.

9. The process of Claim 1, characterized in that the alkaline earth metal tallate dispersion exhibits a bimodal particle size distribution containing two clear maxima.

10. The process of Claim 9, characterized in that one of the maxima is in the range of 10 to 30 microns and the second is from 100 to 150 microns.

## Patentansprüche

1. Verfahren zur Entfernung von Druckfarben aus (zum Deinken von) Druckmedien, das die Stufen umfaßt:
(i) Herstellung einer Zellstoffaufschlämmung, die Druckmedien und Wasser umfaßt,
(ii) Zugabe eines Erdalkalimetalltallats zu der Zellstoffaufschlämmung in einer Menge, die ausreicht, um die Druckfarbe aus der Aufschlämmung zu entfernen und die Helligkeit des Zellstoffs zu erhöhen, und
(iii) Flotations-Deinken der Zellstoffaufschlämmung in einer Flotationszelle,
dadurch gekennzeichnet, daß es sich bei dem Erdalkalimetalltallat um eine Dispersion handelt, die hergestellt worden ist durch Anwendung eines doppelten Verdrängungsverfahrens vor Zugabe derselben zu der Zellstoffaufschlämmung, wobei das doppelte Verdrängungsverfahren umfaßt
(a) die Herstellung einer Voremulsion aus Tallölfettsäuren, Wasser und einem Tensid;
(b) die getrennte Herstellung einer Erdalkalimetallsalz-Dispersion aus einem Erdalkalimetalloxid oder -hydroxid, einer organischen Säure mit einem pKa-Wert in dem Bereich von 2 bis 6 und Wasser und
(c) die Einarbeitung der Voremulsion in die Erdalkalimetallsalz-Dispersion unter hohen Scher- und niedrigen Druck-Bedingungen.

2. Verfahren nach Anspruch 1, dadurch gekennzeichnet, daß das Erdalkalimetalltallat Calciumtallat umfaßt.

3. Verfahren nach Anspruch 1, dadurch gekennzeichnet, daß das Erdalkalimetalltallat in einer Menge von 0,25 bis 2,5 Gew.-% zugegeben wird.

4. Verfahren nach Anspruch 1, dadurch gekennzeichnet, daß das Erdalkalimetalltallat vor dem Flotations-Deinken oder während des Flotations-Deinkens oder sowohl vor als auch während des Flotations-Deinkens zu der Aufschlämmung zugegeben wird.

5. Verfahren nach Anspruch 1, dadurch gekennzeichnet, daß die Voremulsion Tallölfettsäuren enthält, die in teilweise deresiniertem Tallöl enthalten sind.

6. Verfahren nach Anspruch 1, dadurch gekennzeichnet, daß es sich bei der organischen Säure um eine niedere Carbonsäure mit 1 bis 12 Kohlenstoffatomen handelt.

7. Verfahren nach Anspruch 1, dadurch gekennzeichnet, daß die Voremulsion Tallöl-Resinosäuren in einer Menge in dem Bereich von bis zu 10 Gew.-% enthält.

8. Verfahren nach Anspruch 1, dadurch gekennzeichnet, daß die Zellstoffaufschlämmung eine wäßrige Suspension umfaßt, die Zellstofffasern, Druckfarben, Überzüge und anorganische Füllstoffe in einer Menge von 3 bis 18 Gew.-% enthält und daß die Zellstoffaufschlämmung während des Flotations-Deinkens bei einem pH-Wert von 7,5 bis 11 gehalten wird.

9. Verfahren nach Anspruch 1, dadurch gekennzeichnet, daß die Erdalkalimetalltallat-Dispersion eine bimodale Teilchengrößenverteilung aufweist, die 2 klare Maxima enthält.

10. Verfahren nach Anspruch 9, dadurch gekennzeichnet, daß eines der Maxima in dem Bereich von 10 bis 30 µm und das zweite in dem Bereich von 100 bis 150 µm liegen.

## Revendications

1. Procédé d'élimination d'encres de supports imprimés qui comprend les étapes consistant à : (i) former une suspension de pâte comprenant des supports imprimés et de l'eau, (ii) ajouter un tallate de métal alcalino-terreux à la suspension de pâte en une quantité suffisante pour éliminer l'encre de la suspension et pour augmenter la blancheur de la pâte et (iii) soumettre la suspension de pâte à un désencrage par flottation dans une cellule de flottation, caractérisé en ce que le tallate de métal alcalino-terreux est une dispersion préparée par un procédé de double déplacement avant de l'ajouter à la suspension de pâte, dans lequel le procédé de double déplacement consiste à (a) former une pré-émulsion d'acides gras de tallol, d'eau et d'un tensioactif ; (b) former séparément une dispersion d'un sel de métal alcalino-terreux à partir d'un oxyde ou d'un hydroxyde de métal alcalino-terreux, d'un acide organique ayant un pKa s'échelonnant de 2 à 6 et d'eau, et (c) incorporer la pré-émulsion dans la dispersion de sel de métal alcalino-terreux dans des conditions de fort cisaillement et de faible pression.

2. Procédé selon la revendication 1, caractérisé en ce que le tallate de métal alcalino-terreux comprend du tallate de calcium.

3. Procédé selon la revendication 1, caractérisé en ce que le tallate de métal alcalino-terreux est ajouté en une proportion de 0,25 à 2,5 % en poids.

4. Procédé selon la revendication 1, caractérisé en ce que le tallate de métal alcalino-terreux est ajouté à la suspension avant le désencrage par flottation, ou pendant le désencrage par flottation, ou à la fois avant et pendant le désencrage par flottation.

5. Procédé selon la revendication 1, caractérisé en ce que la pré-émulsion contient des acides gras de tallol présents dans du tallol partiellement dépourvu de colophane.

6. Procédé selon la revendication 1, caractérisé en ce que l'acide organique est un acide carboxylique inférieur contenant 1 à 12 atomes de carbone.

7. Procédé selon la revendication 1, caractérisé en ce que la pré-émulsion contient des acides rosinols de tallol en une proportion s'échelonnant jusqu'à 10 % en poids.

8. Procédé selon la revendication 1, caractérisé en ce que la suspension de pâte comprend une suspension aqueuse contenant une fibre de pâte, des encres, des revêtements et des charges minérales, en une proportion de 3 à 18 % en poids, et en ce que la suspension de pâte est maintenue à un pH de 7,5 à 11 pendant le désencrage par flottation.

9. Procédé selon la revendication 1, caractérisé en ce que la dispersion de tallate de métal alcalino-terreux présente une distribution de tailles de particule bimodale contenant deux maxima distincts.

10. Procédé selon la revendication 9, caractérisé en ce que l'un des maxima est dans la gamme de 10 à 30 microns et le deuxième est de 100 à 150 microns.
